# EUROPEAN PATENT APPLICATION

(11) **EP 3 912 617 A1**
(43) Date of publication of application: **24.11.2021**
(21) Application number: 21175015.3
(22) Date of filing: 20.05.2021
(51) Int. Cl.: A61K 9/00, A61K 36/28, A61P 17/00

(54) **DERMATOLOGICAL COMPOSITION COMPRISING HELICHRYSUM OLEOLITE**

(30) Priority: 20.05.2020 IT 202000011746
(71) Applicant: Mocci, Antonio, 09035 Gonnosfanadiga (SU) (IT)
(72) Inventor: Mocci, Antonio, 09035 Gonnosfanadiga (SU) (IT)
(74) Representative: Bianchetti & Minoja SRL

(57) **Abstract**

The present invention relates to a topical composition, useful in the treatment of inflammatory skin disorders, which is characterised by a specific combination of ingredients having a soothing, inflammation-reducing and anti-irritant activity with carriers and excipients having moisturising and emollient properties.

The composition comprises, in weight percent:

- olive oil 20 to 25%

- sweet almond oil 20 to 25%

- Helichrysum oleolite in sweet almond oil 10 to 15%

- vegetable glycerin 10 to 15%

- honey 8 to 12%

- garlic glycol extract 1 to 5%

- Helichrysum oleolite in olive oil 3 to 8%

- beeswax 8 to 15%.

The composition according to the invention has a marked skin protection effect and promotes the permeation of the active agents through the stratum corneum, thereby aiding the repair process.

## Description

The present invention relates to a topical composition which is useful in the treatment of inflammatory skin disorders.

The composition according to the invention combines ingredients having a soothing, inflammation-reducing and anti-irritant activity with carriers and excipients having moisturising and emollient properties. The specific combination of active ingredients with others having a structural function gives rise to a film-forming and skin-protecting effect, and promotes the permeation of the active agents through the stratum corneum, thereby aiding the repair process.

### State of the art

Patent EP915693 describes an oil-in-water emulsion for application to dry or irritated skin which contains lipids, emulsifiers or surfactants in given proportions, and optionally other ingredients including garlic and everlasting flower extracts, hyaluronic acid and aloe vera gel.

Patent application WO2006/081293 relates to anhydrous compositions for topical application to the skin, containing dermatologically active ingredients together with ingredients such as garlic extract, hyaluronic acid, helichrysum oil and aloe vera.

Patent application US2009/0068128 describes a composition for topical application to the skin containing, among other ingredients, glycerol, almond oil, olive oil, beeswax and everlasting extract. The composition is useful for the treatment of skin problems such as scars and rosacea.

### Description of the invention

It has now been found that substances having soothing, inflammation-reducing, anti-irritant and emollient activity, in particular Helichrysum oleolites in sweet almond oil or in olive oil and garlic extract, can be more effectively carried through the stratum corneum when they are combined with a specific mixture of humectants and film-forming agents.

The invention therefore relates to a dermatological composition comprising olive oil, sweet almond oil, Helichrysum oleolite in sweet almond oil, vegetable glycerin, honey, garlic glycol extract, Helichrysum oleolite in olive oil, and beeswax.

Olive oil, preferably refined, and sweet almond oil, perform an emollient and moisturising function. They are present in amounts ranging from 20 to 25% of the total weight of the composition. Sweet almond oil (FU grade) can be obtained by a process of squeezing/cold pressing of the fruit of the almond tree.

Helichrysum oleolite with sweet almonds performs a soothing, anti-irritant function. It is prepared by macerating Helichrysum (everlasting) flowers in sweet almond oil at the ratio of 2:1 by weight, for a time ranging from 7 to 15 days, in particular 10 days, followed by filtration to separate the oily phase from the plant residue. It is present in amounts ranging from 10 to 20% of the total weight of the composition.

Helichrysum oleolite with olive oil performs a soothing, anti-irritant function. It is prepared by macerating Helichrysum (everlasting flower) inflorescences in refined olive oil at the ratio of 2:1 by weight, for a time ranging from 7 to 15 days, in particular 10 days, followed by filtration to separate the oily phase from the plant residue. It is present in amounts ranging from 1 to 10% of the total weight of the composition.

The glycerin is preferably of plant origin, and performs a humectant function. It is present in amounts ranging from 10 to 15% of the total weight of the composition.

The honey is preferably the Millefiori (wildflower) type, and performs an inflammation-reducing and humectant function. It is present in amounts ranging from 7.5 to 12% of the total weight of the composition.

The garlic glycol extract is preferably obtained by extraction from the bulb with propylene glycol at a weight ratio of 1:2. It is present in amounts ranging from 1 to 5% of the total weight of the composition.

Beeswax acts as film-forming agent, and is present in amounts ranging from 8 to 12% by weight.

In a preferred embodiment of the invention, the composition contains (percentages by weight):
Olive oil: 20 to 25%
Sweet almond oil: 20 to 25%
Helichrysum oleolite in sweet almond oil: 10 to 15%
Vegetable glycerin: 10 to 15%
Honey: 8 to 12%
Garlic glycol extract: 1 to 5%
Helichrysum oleolite in olive oil: 3 to 8%
Beeswax: 8 to 15%.

The composition according to the invention naturally takes the form of an ointment with medium-high viscosity. Alternatively, it can be prepared in cream form by adding pharmaceutically acceptable excipients, in particular 15 to 30% of water by weight, and a preservative system, in particular phenoxyethanol and caprylyl glycol, which combine to form a preservative and booster system.

Moreover, hydrophilic matrices such as mucilages or gel can be added to the composition in concentrations of 5% and 10% by weight respectively due to the ingredients of natural derivation present in honey and beeswax at a pH ranging between 5 and 6.5, and can be used as a self-emulsifying base to create physicochemically stable water-in-oil creams.

The composition according to the invention can be prepared by a process comprising the following steps:
1. melting wax and adding olive oil, sweet almond oil and oleolites, to obtain a lipophilic phase;
2. adding honey, garlic extract and any other hydrophilic ingredients to the lipophilic phase under turboemulsion;
3. when the ingredients have been added, leaving under slow stirring for at least 2 hours to cool and removing air bubbles, or operating under vacuum.

The composition according to the invention is indicated in general for the treatment of inflammatory skin disorders, especially those characterised by thickening of the stratum corneum, flaking, redness and itching.

The formulation characteristics, due to a skin perspiration regulating mechanism, allow both deep hydration of thickened keratinocytes, with a consequent increase in their permeability, and maintenance/reinstatement of skin homeostasis conditions, which aids the repair process. Due to said characteristics, the formulation is suitable for the treatment of various kinds of inflammatory skin disorders. The composition constitutes a self-preserving and self-emulsifying system despite the fact that no structural excipients designed for said purpose are present in its composition.

The clinical evidence obtained, and the results of the in vitro transdermal tests, demonstrate a synergic action produced by the specific combination of active ingredients, carriers and excipients selected to promote the action and penetration of the active ingredient through the skin.

### Examples

### Example 1.

An ointment having the following percentage composition by weight was prepared:

| | |
|---|---|
| Olive oil | 22.82% |
| Sweet almond oil | 22.82% |
| Helichrysum oleolite in sweet almond oil | 13% |
| Vegetable glycerin | 13.78% |
| Honey | 10% |
| Garlic glycol extract | 3% |
| Helichrysum oleolite in olive oil | 4.27% |
| Beeswax | 10.79% |

### Characterisation of formulation

A number of tests were conducted to verify the characteristics of the formulation in terms of physicochemical stability and properties.

**Table 1**

| | |
|---|---|
| Colour | Yellowish |
| Odour | Characteristic (garlic extract) |
| Appearance | Homogeneous |
| pH | 5.7 |
| Rheological behaviour | Non-Newtonian, pseudoplastic |
| Viscosity at 25°C* | 29800 cP (BrooKfield RV 7) |
| Coal escence/efflorescence | None |
| Density | 0.98 g/ml |

Microbiological behaviour:

**Table 2**

| | UFC/g | | | | |
|---|---|---|---|---|---|
| | Staphylococcus aureus ATCC 6538 | Pseudomonas aeruginosa ATCC 9027 | Escherichia coli ATCC 8739 | Candida albicans ATCC10231 | Aspergillus brasiliensis ATCC 16404 |
| T0 | 1300000 | 800000 | 2000000 | 27000 | 17000 |
| T7 | 480 | 2200 | 18000 | <10 | NP |
| T14 | <10 | <10 | <10 | <10 | 60 |
| T28 | <10 | <10 | <10 | <10 | 10 |

**Table 3**

| | RIDUZIONE LOG10/LOG10 REDUCTION | | | | |
|---|---|---|---|---|---|
| | Staphylococcus aureus ATCC 6538 | Pseudomonas aeruginosa ATCC 9027 | Escherichia coli ATCC 8739 | Candida albicans ATCC10231 | Aspergillus brasiliensis ATCC 16404 |
| T0 | - | - | - | - | - |
| T7 | 3,4 | 2,6 | 2,0 | 4,4 | NP |
| T14 | 6,1 | 5,9 | 6,3 | 4,4 | 2,5 |
| | NI | NI | NI | NI | NI |
| T28 | 6,1 | 5,9 | 6,3 | 4,4 | 3,2 |
| | NI | NI | NI | NI | NI |

### Example 2 - Skin test

A study was conducted on the ointment in question to evaluate its compatibility with human skin (irritant potential) under both normal conditions of use and reasonably foreseeable conditions of misuse, in compliance with the latest recommendations of the Helsinki Declaration (64th WMA General Assembly, Fortaleza, Brazil, October 2013) and in accordance with the "Guidelines for the Assessment of Skin Tolerance of Potentially Irritant Cosmetic Ingredients", Edition of 1997 - https://www.cosmeticseurope.eu/publications-cosmetics-europe-association).

For this purpose, the product was used in a single application, in an occlusive patch test, on the intact skin of the backs of 20 volunteers of both sexes aged between 18 and 65 years. Said test does not rule out the allergenic potential of the product tested.

### Method

The product under test was left in contact with the skin for 48 hours using an occlusive patch (model Curatest® F, patch test strip, Lohmann and Rauscher International GMBH and Co., Rengsdorf, Germany) in a sufficient amount to fill a reaction disc with an area of 1 cm² (approximately 0.07-0.1 ml). The evaluation is always conducted in parallel with a negative control, prepared as follows: 1) if the product is tested pure: empty patch; 2) if the product is diluted: patch with about 0.07-0.1 ml of water or mineral oil (depending on the solvent used).

Fifteen minutes and 24 hours after the removal of the occlusive device, the skin reactivity to the test (commonly described as the "patch test") was evaluated by analysing the following parameters: erythema, flaking, swelling and blistering.

The test was conducted on a single-blind basis, under the clinical direction of a doctor specialising in allergology having proven experience in clinical trials, and supervised by a doctor specialising in dermatology.

At the various observation times (15 min and 24 h after removal of the patch), the area was carefully examined according to the parameters set out in Table 4. Each parameter was evaluated on a scale of 0 to 3 to score the various severity levels of the reactions observed. All other adverse local and systemic effects, even if purely subjective, were recorded.

**Table 4**

| **Erythema** | **Swelling** | **Dryness/flaking** | **Blisters** | **Score** |
|---|---|---|---|---|
| None | None | No sign | None | 0 |
| Slight erythema, scarcely visible | Slight swelling (edges of lesion raised and clearly defined) | Dryness and slight flaking (smooth skin) | Very small (scarcely visible) | 1 |
| Modest, uniform redness | Moderate swelling (edges of lesion raised by about 1 mm) | Moderate flaking | Small but clearly visible, with clearly defined edges | 2 |
| Severe, uniform redness (with or without broken skin and/or scabs) | Severe swelling (wound edges raised by over 1 mm and extending beyond the area exposed to the product) | Severe flaking | Large, clearly defined | 3 |

The scores allocated for the parameters considered (erythema, flaking, swelling and blisters) were recorded for each volunteer. The data were considered cumulatively for the entire group of volunteers, and the average was calculated. This allowed the extrapolation of the Mean Irritation Index (MII), which classifies the product according to the value ranges specified in the table below (Table 5):

**Table 5**

| **MII** | **CLASSES** |
|---|---|
| 0.4 | Non-irritating |
| 0.5 ≤ MII ≤ 1.9 | Slightly irritating |
| 2.0 ≤ MII ≤ 4.9 | Moderately irritating |
| 5.0 ≤ MII ≤ 8.0 | Strongly irritating |

### Results

**Table 6**

| | **CONCENTRATION** | **MII 15 minutes after removal of patch** | **MII 24 hours after removal of patch** |
|---|---|---|---|
| PRODUCT TESTED (Ointment) | Pure | 0 | 0 |
| CONTROL | Empty | 0 | 0 |

The calculation of the Mean Irritation Index (MII), under the experimental conditions used, allowed the product under test to be declared non-irritating.

### Example 3 - Clinical test

The efficacy of the composition was evaluated in a study conducted on paediatric patients suffering from skin disorders (subject to obtaining informed consent).

The patient was treated with the above formulation for 5 consecutive days. At the start of the treatment the patient's skin was strongly irritated and red due to the inflammation, which gradually regressed until it had completely disappeared after 5 days' treatment.

## Claims

1. A dermatological composition comprising, in weight percent:
- olive oil 20 to 25%
- sweet almond oil 20 to 25%
- Helichrysum oleolite in sweet almond oil 10 to 15%
- vegetable glycerin 10 to 15%
- honey 8 to 12%
- garlic glycol extract 1 to 5%
- Helichrysum oleolite in olive oil 3 to 8%
- beeswax 8 to 15%.

2. The composition of claim 1 comprising, in weight percent:
- olive oil 22.82%
- sweet almond oil 22.82%
- Helichrysum oleolite in sweet almond oil 13%
- vegetable glycerin 13.78%
- honey 10%
- garlic glycol extract 3%
- Helichrysum oleolite in olive oil 4.27%
- beeswax 10.79%.

3. The composition of claims 1-2, wherein, independently of one another:
- the sweet almond oil is obtained by squeezing/cold pressing of the fruit of the almond tree;
- the Helichrysum oleolite in sweet almond oil is obtained by maceration of Helichrysum inflorescences in sweet almond oil in a 2:1 weight ratio, for a time ranging from 7 to 15 days, preferably 10 days, and subsequent filtration to separate the oily phase from the vegetable residue;
- the Helichrysum oleolite in olive oil is obtained by maceration of Helichrysum inflorescences in refined olive oil in a 2:1 weight ratio for a time ranging from 7 to 15 days, preferably 10 days, and subsequent filtration to separate the oily phase from the vegetable residue;
- the garlic glycol extract is obtained by extraction from the bulb with propylene glycol in a weight ratio of 1:2.

4. The composition of claims 1-3, which is in the form of an ointment or cream.

5. The composition of claims 1-4, for use in the treatment of inflammatory skin disorders.

6. The composition for use according to claim 5, wherein said inflammatory skin disorder is **characterised by** thickening of the stratum corneum, flaking, redness and itching.
